# EUROPÄISCHE PATENTSCHRIFT

(11) **EP 0 664 454 B1**
(45) Veröffentlichungstag und Bekanntmachung des Hinweises auf die Patenterteilung: **04.04.2001**
(21) Anmeldenummer: 95100521.4
(22) Anmeldetag: 17.01.1995
(51) Int. Cl.: G01N 33/84, G01N 31/22

(54) **Verfahren und Reagenz zur Bestimmung von Eisen**
Method and reagent for the determination of iron
Méthode et réactif pour la détermination du fer

(30) Priorität: 21.01.1994 DE 4401754
(43) Veröffentlichungstag der Anmeldung: 26.07.1995
(60) Teilanmeldung: 00115514.2
(73) Patentinhaber: Roche Diagnostics GmbH, 68298 Mannheim (DE)
(72) Erfinder: Weisheit, Ralph, Dr., D-82362 Weilheim (DE); Luz, Renate, D-82327 Tutzing (DE)

(56) Entgegenhaltungen:
- EP-A- 0 130 537
- EP-A- 0 306 859
- EP-A- 0 343 592
- EP-A- 0 404 590

## Beschreibung

Die Erfindung betrifft ein Verfahren zur Bestimmung von Eisen im Serum unter Freisetzung des gebundenen Eisens durch Zusatz eines Protein-Denaturierungsmittels, Reduktion des freigesetzten Eisens zu Fe²⁺, Zugabe einer Farbreagenzlösung und photometrische Messung des gebildeten Farbkomplexes sowie eine Reagenzkombination, die auch ohne Zusatz von aufhellenden Tensidmischungen für die Messung von lipämischen Seren geeignet ist.

Eisenstoffwechselstörungen, insbesondere Eisenmangel und Eisenresorptionsstörungen sind vor allem in der weiblichen Bevölkerung weit verbreitet. Der Nachweis von Eisen in Körperflüssigkeiten, insbesondere im Serum gehört daher zu den Standardbestimmungen in der medizinischen Analytik. Eisen wird mit der Nahrung zugeführt und über die Schleimhaut des Darmes aufgenommen. An Transferrin in 3-wertigem Zustand gebunden wird es dann zum Knochenmark transportiert, wo es hauptsächlich in das Hämoglobin eingebaut wird. Wird zu wenig Eisen aufgenommen, so kommt es zu anämischen Erscheinungen.

Die Bestimmung des Eisens im Serum gehört zu den am häufigsten durchgeführten Spurenelementanalysen in der klinischen Diagnostik. Hierzu sind verschiedene Verfahren bekannt. So ist in Clin. Chem. 26 (1980) 327 - 331 ein Verfahren beschrieben, bei dem das an Transferrin in Form eines Carbonatkomplexes gebundene 3-wertige Eisen in stark saurem Milieu freigesetzt wird. Nachteil dieses Verfahrens ist es, daß die stark sauren Reagenzien ätzende und korrodierende Eigenschaften aufweisen. Um diesen Nachteil auszuräumen, ist es beispielsweise aus Clin. Chem. 23 (1977) 237 - 240 und aus Z. Clin. Chem. 3 (1965) 96 - 99 bekannt, proteindenaturierende Agenzien, wie konzentriertes Guanidiniumchlorid oder anionische Detergenzien, zur Freisetzung des Eisens zu verwenden.

In den bekannten Ausführungsformen dieser Verfahren treten bei der Bestimmung von Eisen in trüben lipämischen Seren Fehlmessungen auf. Weder Guanidiniumchlorid noch die anionischen Detergenzien besitzen eine ausreichende Aufklarungskraft, um eine vollständige Beseitigung der Trübung zu erreichen.

Zur Lösung dieses Problems wurde in der EP-A-0 130 537 vorgeschlagen, eine Mischung aus nicht-ionischen und anionischen Detergenzien zur Freisetzung des Eisens im schwach sauren Medium zu verwenden. Es wurde jedoch gefunden, daß bei Verwendung dieser Detergenzien zwar rasch und vollständig eine Aufklarung von lipämischen Seren erfolgt, aber für Seren mit einem erhöhten Gehalt an Immunglobulin (Gammopathie-Seren) eine Eintrübung beobachtet wird, die das Meßergebnis verfälschen kann. Zudem ist die Wiederfindung bei stark hämolytischen Seren nicht völlig befriedigend.

In der EP-A-0 343 592 wird zur Lösung dieses Problems vorgeschlagen, daß man das Serum mit einer ersten Lösung eines Denaturierungsmittels in einer Konzentration von 1 - 8 mol/l versetzt, nach Zugabe der ersten Lösung eine Trübungsmessung vornimmt, danach eine zweite Lösung zusetzt, die das Farbreagenz und ebenfalls ein Denaturierungsmittel in einer Konzentration von 1 - 8 mol/l enthält, und danach den Farbkomplex bestimmt und zur Ermittlung der Eisenmenge den Trübungsmeßwert vom Meßwert der Farbkomplexlösung subtrahiert. Ein Nachteil dieser Methode ist, daß es in der zweiten Lösung zu Ausfällungen, die wahrscheinlich aus einem Komplex des Farbstoffes und des Denaturierungsmittels bestehen, kommen kann. Daher muß die Konzentration des Farbstoffes in dieser Lösung abgesenkt werden, wodurch es zu Störungen durch EDTA-Kontaminationen und Falschmessungen durch einen nicht linearen Reaktionsverlauf bei Proben mit niedrigem Eisengehalt kommen kann.

Es bestand daher die Aufgabe, ein Verfahren und ein Reagenz zur Bestimmung von Eisen in einer biologischen Probe zu schaffen, bei dem einerseits keine aggressiven Bestandteile verwendet werden müssen und andererseits eine Störung durch lipämische Seren vermieden werden kann, ohne daß die Messung durch hämolytische Proben oder Seren mit erhöhtem Immunglobulingehalt gestört wird. Aus Gründen der Automatisierbarkeit und Rationalisierung ist es ferner wünschenswert, daß keine vorhergehende Enteiweißung der Probe erforderlich ist.

Diese Aufgabe wird erfindungsgemäß gelöst durch ein Verfahren zur Bestimmung von Eisen in einer biologischen Probe unter Freisetzung des gebundenen Eisens durch Zusatz eines Protein-Denaturierungsmittels, Reduktion des freigesetzten Eisens zu Fe²⁺, Zugabe einer Farbreagenzlösung und photometrische Messung des gebildeten Farbkomplexes, das dadurch gekennzeichnet ist, daß man die biologische Probe mit einem Harnstoff- oder Härnstoffderivat-haltigem Denaturierungsmittel und einem Fettalkoholpolyglykolether versetzt. Als biologische Probe wird bevorzugt Blutserum eingesetzt. Neben Serum kann auch Plasma, vorzugsweise Heparin-Plasma, eingesetzt werden.

Die Erfindung beruht auf der Feststellung daß durch die Kombination eines Harnstoff- oder Harnstoffderivat-haltigen Denaturierungsmittels und einem einzigen, ganz bestimmten Detergenz - einem Fettalkoholpolyglykolether welcher mehr als 4 und weniger als 7 Ethoxy-Einheiten, insbesondere 5 bis 6 Ethoxy-Einheiten enthält - eine Eintrübung beim Zusatz des Farbreagenzes vermieden werden kann. Damit ist eine störungsfreie Messung sowohl von lipämischen Seren als auch von Gammopathie-Seren möglich. Zur Vermeidung von Eintrübungen, die bei lipämischen Seren auftreten, ist es nicht mehr notwendig, Detergenzkombinationen, wie in der EP-A-0 130 537, DE-A-27 24 757 oder DE-A-37 29 502 beschrieben, zu verwenden.

Zur Ablösung des Eisens von Bindeproteinen, insbesondere von Ferritin, ist der Zusatz von verschiedenen Protein-Denaturierungsreagenzien bekannt. Zur Proteindenaturierung im Rahmen der Erfindung geeignet sind Harnstoff oder Harnstoffderivate wie beispielsweise Salze des Kations Guanidinium, besonders bevorzugt die Salze Guanidiniumchlorid-, -acetat, -thiocyanat oder -sulfat.

Das Denaturierungsmittel wird in einer ersten Lösung des erfindungsgemäßen Eisentest in Kombination mit dem Fettalkoholpolyglykolether verwendet. Die Konzentration des Denaturierungsmittels in der ersten Lösung des erfindungsgemäßen Eisentests muß relativ hoch sein und zwischen 1 und 8 mol/l liegen. Beste Ergebnisse liefern Konzentrationen zwischen 4 und 6 mol/l. Bei Konzentrationen von unter 1 mol/l wird die Ablösung des Eisens von Transferrin zunehmend langsamer. Für das besonders bevorzugte Guanidiniumchlorid liegt der bevorzugte Konzentrationsbereich zwischen 1 bis 6 mol/l, besonders bevorzugt zwischen 4 und 5 mol/l.

Als Fettalkoholpolyglykolether werden solche verwendet, deren Ethoxylierungsgrad mehr als 4 und bis zu 7 Ethoxy-Einheiten aufweisen und zu deren Herstellung ein verzweigter oder unverzweigter Fettalkohol verwendet wurde. Bevorzugt wird ein Fettalkohol mit im Mittel 9 - 11 Kohlenstoffatomen verwendet. Besonders bevorzugte Fettalkoholpolyglykolether sind beispielsweise Lutensol^{®} ON 50 (Decylpolyethylenglykolether mit durchschnittlich 5 Ethoxy-Einheiten), Lutensol^{®} ON 60 (Decylpolyethylenglykolether mit durchschnittlich 6 Ethoxy-Einheiten), Imbentin^{®}-C/91/35 (Alkylpolyethylenglykolether mit 9 bis 11 C-Atomen im Alkylrest und 5 Ethoxy-Einheiten im Polyethylenglykol-Rest), Imbentin^{®}-AG/100/050 (Decylpolyethylenglykolether mit durchschnittlich 5 Ethoxy-Einheiten). Unter der Angabe des Ethoxylierungsgrades wird der durchschnittliche Ethoxylierungsgrad einer Charge verstanden, da es sich bei den Fettalkoholpolyglykolethern meist um Gemische aus Substanzen mit leicht unterschiedlichem Ethoxylierungsgrad handelt.

Die Konzentration des Fettalkoholpolyglykolethers in der ersten Lösung des erfindungsgemäßen Eisentests muß mindestens 10 g/l betragen. Bevorzugt beträgt die Konzentration mindestens 30 g/l, da hierbei eine schnellere Aufklarung lipämischer Proben auftritt. Die obere Konzentration des Fettalkoholpolyglykolethers ergibt sich durch die technisch bedingte Obergrenze, das heißt, die Konzentration, bei der das Detergenz nicht mehr vollständig in Lösung geht. Besonders bevorzugt ist eine Konzentration des Detergenzes von 50 bis 100 g/l. Durch die Verwendung der genannten Fettalkoholpolyglykolether ist es möglich, eine vollständige Aufklarung lipämischer Proben innerhalb weniger Minuten zu erreichen. Meist reichen fünf Minuten zur Aufklarung aus. Damit ist das Reagenz auch für schnell messende Analysenautomaten verwendbar.

Tenside mit niedrigerem oder höherem Etoxylierungsgrad als die erfindungsgemäß verwendeten Fettalkoholpolyglykolether, wie beispielsweise Tween^{®}20, Genapol X-080, Oxetal ID 104, Thesit^{®}, Lutensol^{®}ON 30, Triton^{®}X-100, sind zur Aufklarung lipämischer Proben in einem Harnstoff- oder Harnstoffderivat-haltigen Reagenz nicht geeignet.

Zur Durchführung des erfindungsgemäßen Verfahrens wird die Probelösung bevorzugt in einem schwach sauren Bereich, besonders bevorzugt im Bereich zwischen pH 3,5 und pH 6 gepuffert. Als Puffersubstanzen sind Verbindungen geeignet, die einen pK-Wert von 3,5 bis 6 aufweisen und Eisen nicht komplexieren. Geeignet sind beispielsweise Acetat-, Phosphat- oder Succinatpuffer. Eine weitere bevorzugte Variante besteht darin, zur Freisetzung des Eisens zunächst einen sauren pH-Wert einzustellen, besonders bevorzugt pH 2 bis pH 6, und danach auf einen pH-Wert umzupuffem, der mit dem zum Nachweis des Eisens gewählten Farbsystem eine optimale Farbausbeute ergibt. Die hierzu geeigneten pH-Werte sind dem Fachmann an sich bekannt beispielsweise aus Fielding, Methods in Hematology 1(Iron), 15-49 (1980).

Bevorzugt wird als Puffersubstanz Acetatpuffer eingesetzt. Der Puffer wird bevorzugt in einer Konzentration von 20 bis 500 mmol/l, insbesonders bevorzugt 50 bis 150 mmol/l, verwendet. Die Puffersubstanz kann in der ersten Lösung des erfindungsgemäßen Eisentest, die das Denaturierungsmittel und den Fettalkoholpolyglykolether enthält, enthalten sein. Zusätzlich können auch die weiteren Lösungen oder die weitere Lösung des erfindungsgemäßen Eisentests gepuffert sein.

Zur Bestimmung des Eisens wird in an sich bekannter Weise der Probelösung ein Reduktionsmittel, wie zum Beispiel Ascorbinsäure, Dithionit oder Hydroxylamin, zugesetzt, um das in 3-wertiger Form vorliegende freigesetzte Eisen in die 2-wertige Form zu reduzieren. Das Reduktionsmittel kann sowohl in der ersten Lösung zusammen mit dem Denaturierungsmittel als auch in der zweiten Lösung, die weiterhin das für den Nachweis von Eisen geeignete Farbsystem enthält, zugesetzt werden. Bevorzugt wird das Reduktionsmittel in der zweiten Lösung zugesetzt.

Farbsysteme zum Nachweis von Eisen sind zum Beispiel beschrieben in der EP-A-0 228 060, Clin. Biochem. 14 (1981), 311 - 315 und Clin. Chem. 23 (1979), 237 - 240. Besonders sind Komplexbildner vom Ferrointyp geeignet, die mit Eisen einen Farbstoff liefern, der photometrisch ausgewertet werden kann. Als geeignete Substanzen sind zum Beispiel Bathophenanthrolin, Feren (3-(2-pyridyl)-5,6-bis(2-[furylsulfonsäure])-1,2,4-triazin-dinatriumsalz) und Ferrozin (3'(2'-Pyridyl)-5,6-diphenyl-1,2,4-triazin-sulfonsäure-dinatriumsalz) zu nennen. Die Farbstoffbildung erfolgt dabei proportional zum Eisengehalt der Probe und kann in an sich bekannter Weise photometrisch ausgewertet werden.

Mit dieser Erfindung ist es erstmals gelungen, unter Verzicht auf ein ganzes Detergenzsystem und durch Verwendung nur eines einzigen Tensides ein Reagenz herzustellen, das für die photometrische Bestimmung von Eisen auch in stark lipämischen biologischen Proben geeignet ist.

Ein weiterer Gegenstand der Erfindung ist daher die Verwendung eines Gemisches eines Harnstoff- oder Harnstoffderivat-haltigen Denaturierungsmittels und eines Fettalkoholpolyglykolethers zur Verhinderung von Lipämie- und Gammopathie-Störungen bei der photometrischen Bestimmung von Eisen in einer Probe.

Die Überprüfung der Wirksamkeit des Reagenzes zur Aufklarung lipämischer Proben kann photometrisch bei einem Probe-/Reagenzverhältnis von 1:5 bis 1:25, bevorzugt 1:10 bis 1:15, erfolgen. Dabei wird als vollständige Aufklarung definiert, wenn eine Lösung aus Intralipid®20% (Kabi Pharmacia GmbH, Deutschland), eine Fettemulsion auf Sojabohnenölbasis, die mit Wasser im Verhältnis 1:20 weiter verdünnt wurde, bei 37°C, einer Wellenlänge von 578 nm und einer Schichtdicke von 1 cm gegen die Reagenzlösung als Leerwert gemessen, nach fünf Minuten eine Extinktion von maximal 5 mE ergibt.

Die Aufklarung kann aber auch an lipämischen biologischen Proben durch Messung des Extinktion/Zeit (E/t)-Verlaufes geprüft werden, wobei sie dann definitionsgemäß als vollständig bezeichnet wird, wenn die nach fünf Minuten gemessene Extinktion sich von der nach 10 Minuten gemessenen Extinktion um weniger als 5 mE, bevorzugt kleiner 2 mE, unterscheidet.

Für die Beurteilung der Lipämie-Störung wird der Eisengehalt einer mit Intralipid ^{®}20 % aufgestockten biologischen Probe mit dem der gleichen, jedoch mit Wasser versetzten Probe verglichen (analog Glick, Clin. Chem. 32 (1986), 470-475). Eine Lipämie-Störung liegt dann vor, wenn sich der gemessene Eisengehalt einer Mischung aus einem Volumenteil Intralipid^{®}20 % und 19 Volumenteilen Serum und der gemessene Eisengehalt einer Mischung aus einem Volumenteil Wasser und 19 Volumenteilen des gleichen Serums um mehr als 5 µg/dl unterscheidet.

Ein weiterer Gegenstand der Erfindung ist eine Reagenzienkombination zur Bestimmung von Eisen im Serum, gekennzeichnet durch ein erstes Reagenz, enthaltend 1 bis 8 mol/l Denaturierungsmittel und mindestens 10 g/l Fettalkoholpolyglykolether und getrennt davon ein zweites Reagenz, enthaltend 0,5 bis 50 mmol/l Farbstoff in Form wässriger Lösungen oder zu deren Herstellung geeignete Trockengemische. Beide Reagenzien oder auch nur das erste Reagenz können zusätzlich 20 bis 500 mmol/l Puffersubstanz enthalten.

Das zweite Reagenz kann zusätzlich bereits ein Reduktionsmittel, beispielsweise Ascorbinsäure, Dithionit oder Hydroxylamin, in einer Konzentration von 40 bis 200 mmol/l enthalten Das Reduktionsmittel kann auch in höherer Konzentration (z. B. 1000 mmol/l) enthalten sein. Jedoch besteht dann die Gefahr, daß bei Langzeitlagerung schwerlösliche Abbauprodukte (z. B. Oxalat aus Ascorbinsäure) gebildet werden, die zu Reagenztrübungen führen können. Überraschenderweise hat sich herausgestellt, daß das Reduktionsmittel in dem zweiten Reagenz in Form einer wässrigen Lösung stabil ist. In den bisher üblichen Reagenzienkombinationen zur Bestimmung von Eisen im Serum mußte das Reduktionsmittel meist kurz vor der Verwendung dem Flüssigreagenz zugefügt werden.

Die folgenden Beispiele erläutern die Erfindung näher.

### Beispiel 1

Zur Eisenbestimmung in Serum werden folgende Reagenzien eingesetzt:

| **Reagenz 1:** | |
|---|---|
| 4 mol/l | Guanidiniumhydrochlorid |
| 0,15 mol/l | Acetatpuffer pH 5,0 |
| 0,100 mol/l | Thioharnstoff |

Fettalkoholpolyglykolether in der jeweils weiter unten angegebenen Konzentration

| **Reagenz 2:** | |
|---|---|
| 15 mmol/l | Ferrozin |
| 0,1 mol/l | Ascorbinsäure |

Die Versuchsdurchführungen wurden an einem Hitachi 717^{TM} Spektrophotometer durchgeführt.

20 µl Probe wurden mit 250 µl Reagenz 1 gemischt. Nach 4,6 Minuten Inkubation bei 37°C wurden 50 µl Reagenz 2 hinzupipettiert. Die Extinktion 1 wurde kurz vor Zugabe des Reagenzes 2 bestimmt. Die Messung der Extinktion 2 erfolgte nach 6 Minuten. Hieraus kann die Extinktionsdifferenz ΔE = E2 - E1 bestimmt werden. Alternativ hierzu wurden zur besseren Darstellung der Aufklarung E/t-Diagramme aufgezeichnet.

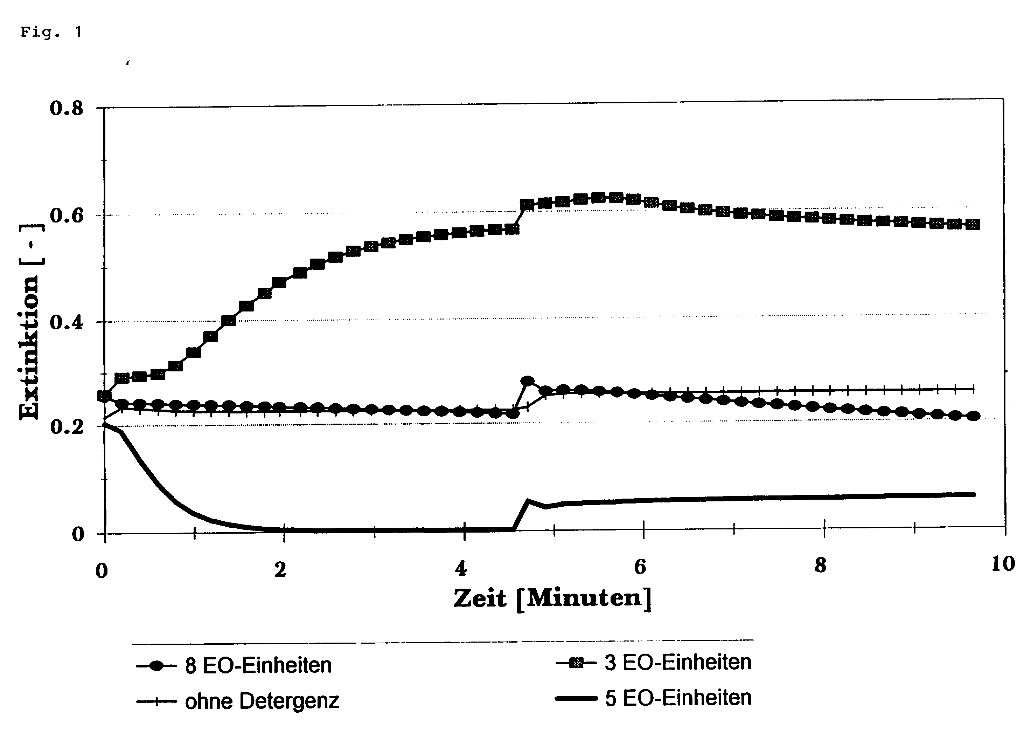

Im ersten Beispiel wurde die Aufhellung mit verschiedenen Detergenzien im Reagenz 1 am Beispiel eines Humanserums, das mit Intralipid^{®}20 aufgestockt war, anhand von E/t-Diagrammen ermittelt. Der Triglycerid-Gehalt der aufgestockten Humanserumprobe betrug ca. 2000 mg/dl. Als Detergenzien wurden Fettalkoholpolyglykolether mit 3 Ethoxy-Einheiten (Lutensol^{®}ON 30), 5 Ethoxy-Einheiten (Lutensol^{®}ON 50) und 8 Ethoxy-Einheiten (Genapol X-080) eingesetzt. Als Kontrolle wurde Reagenz 1 ohne Detergenz verwendet. Die Ergebnisse sind in der Figur 1 dargestellt. Es ist klar zu erkennen, daß lediglich das erfindungsgemäße Reagenz mit einem Fettalkoholpolyglykolether von 5 Ethoxy-Einheiten zu einer Aufhellung der lipämischen Probe führt. Bereits nach ca. zwei Minuten ist die Extinktion auf den Nullwert abgesunken. Ein Fettalkoholpolyglykolether mit 8 Ethoxy-Einheiten führt zu keiner Aufhellung der Probe. Der Fettalkoholpolyglykolether mit 3 Ethoxy-Einheiten führt sogar zu einer verstärkten Eintrübung der Probe.

### Beispiel 2

Die Versuchsdurchführung wurde analog dem Beispiel 1 durchgeführt. Es wurden verschiedene erfindungsgemäße Fettalkoholpolyglykolether mit 4,5 bis 6 Ethoxy-Einheiten eingesetzt (Imbentin-E/V-177: 4,5 Ethoxy-Einheiten; Lutensol^{®} ON 50: 5 Ethoxy-Einheiten; Imbentin-AG/100/50: 5 Ethoxy-Einheiten; Imbentin-C/91/35: 5 Ethoxy-Einheiten; Lutensol^{®} ON60: 6 Ethoxy-Einheiten).

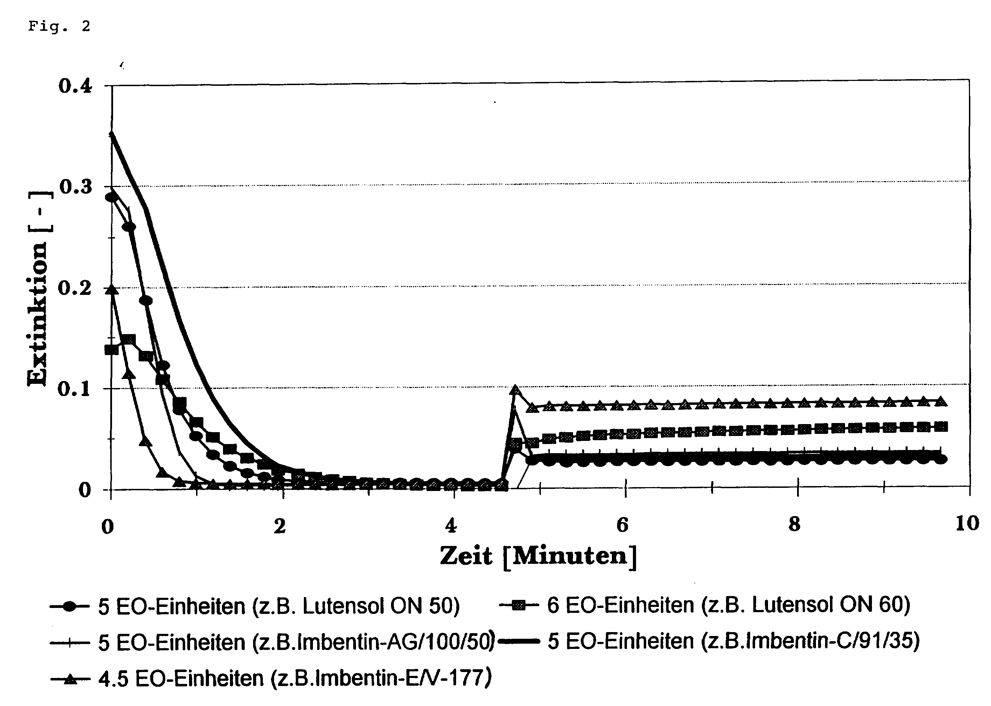

Die Ergebnisse sind in der Figur 2 dargestellt. Wie deutlich zu erkennen ist, führen alle erfindungsgemäßen Fettalkoholpolyglykolether bei einem Humanserum, das mit Intralipid aufgestockt wurde (Triglycerid-Gehalt ca. 2000 mg/dl), zu einer schnellen Aufhellung der Probe. Alle Proben waren nach ca. zwei Minuten aufgehellt. Imbentin E/V-177 und Imbentin-AG/100/50 führten bereits nach einer Minute zu einer vollständigen Aufhellung der Probe.

### Beispiel 3

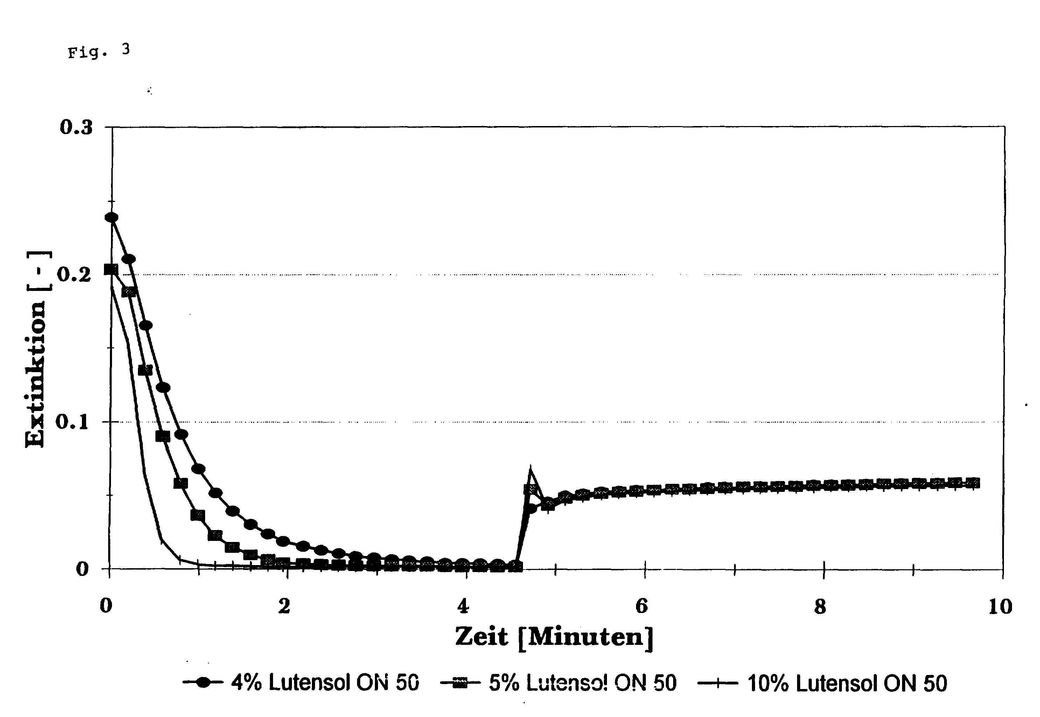

Am Beispiel Lutensol^{®} ON 50 wurde der Einfluß der Konzentration des Detergenz auf die Aufhellung von Humanserum, welches mit Intralipid bis zu einem Triglycerid-Gehalt von ca. 2000 mg/dl aufgestockt war, ermittelt. Die Ergebnisse sind in der Figur 3 dargestellt. Eine Konzentration von 4 % Lutensol^{®} ON 50 führt nach ca. drei Minuten zu einer vollständigen Aufklarung der Probe, 5 % Lutensol^{®} ON 50 führen bereits nach ca. zwei Minuten zu einer vollständigen Aufklarung und 10 % Lutensol^{®} ON 50 bereits nach 1 Minute zu einer vollständigen Aufklarung der Probe.

Wie deutlich zu erkennen ist, führt auch die sehr hohe Konzentration von 10 % zu keiner Beeinflussung der Farbentwicklung nach Zusatz des Reagenz 2.

Die Versuchsdurchführung erfolgte analog dem Beispiel 1.

### Beispiel 4

An diesem Beispiel wurde gezeigt, daß nicht nur artifizielle mit Intralipid aufgestockte Proben mit dem erfindungsgemäßen Reagenz aufgeklart werden können, sondern auch natürlich vorkommende lipämische Humanseren. In diesem Beispiel wurde lipämisches Humanserum verwendet, das einen Triglycerid-Gehalt von ca. 1000 mg/dl enthielt. Die Versuchsdurchführung erfolgte analog dem Beispiel 1. Im Reagenz 1 waren als Detergenz 5 % Lutensol^{®} ON 50 enthalten.

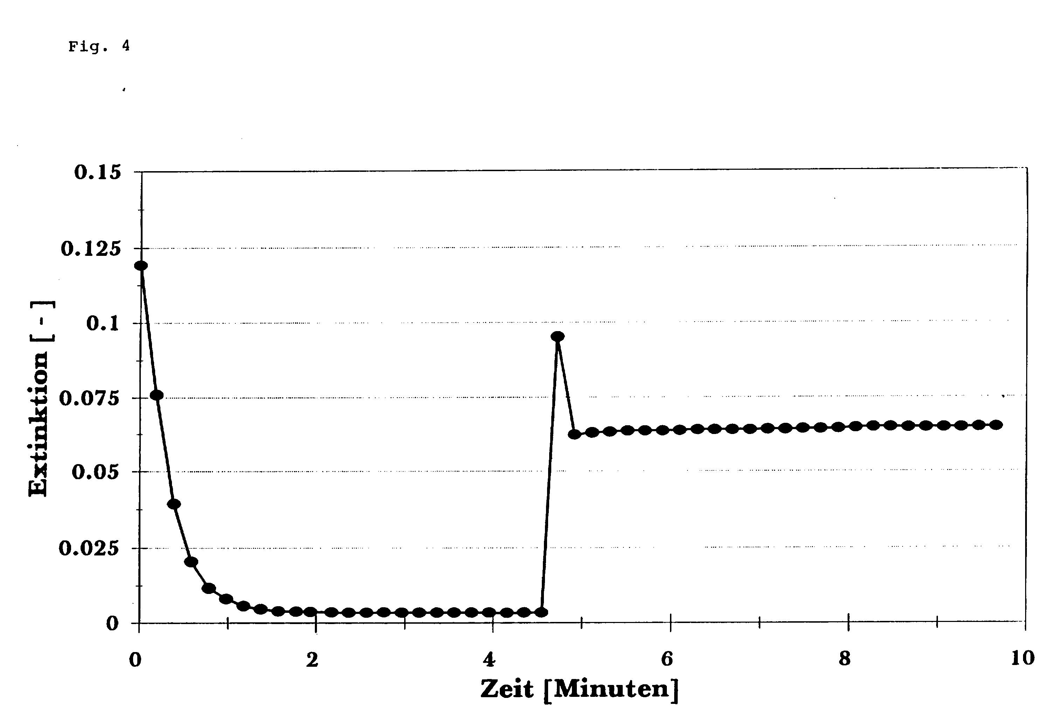

Wie aus der Figur 4 eindeutig zu erkennen ist, führt die Zugabe des Reagenzes 1 bereits nach zwei Minuten zu einer vollständigen Aufklarung des lipämischen Humanserums.

## Patentansprüche

1. Verfahren zur Bestimmung von Eisen in Serum unter Freisetzung des gebundenen Eisens durch Zusatz eines Protein-Denaturierungsmittels, Reduktion des freigesetzten Eisen zu Fe²⁺, Zugabe einer Farbreagenzlösung und photometrische Messung des gebildeten Farbkomplexes, dadurch gekennzeichnet, daß man das Serum mit einem Harnstoff- oder harnstoffderivathaltigem Denaturierungsmittel und einem Fettalkoholpolyglykolether versetzt, welcher mehr als 4 und weniger als 7 Ethoxy-Einheiten, insbesondere 5 bis 6 Ethoxy-Einheiten enthält.

2. Verfahren nach einem der vorhergehenden Ansprüchen, dadurch gekennzeichnet, daß der Fettalkoholpolyglykolether einen verzweigten oder unverzweigten Fettalkoholrest mit bevorzugt 9 - 11 Kohlenstoffatomen enthält.

3. Verfahren nach einem der vorhergehenden Ansprüche, dadurch gekennzeichnet, daß der Fettalkoholpolyglykolether in einer Konzentration von mindestens 10, insbesondere 50 bis 100 g/l, verwendet wird.

4. Verfahren nach einem der vorhergehenden Ansprüche, dadurch gekennzeichnet, daß als Denaturierungsmittel ein Guanidiniumsalz verwendet wird.

5. Verfahren nach einem der vorhergehenden Ansprüche, dadurch gekennzeichnet, daß man zur Reduktion des Eisens Ascorbinsäure, Dithionit oder Hydroxylamin verwendet.

6. Verfahren nach einem der vorhergehenden Ansprüche, dadurch gekennzeichnet, daß man als Farbreagenz Bathophenanthrolin, Feren oder Ferrozin verwendet.

7. Verwendung eines Gemisches eines Harnstoff- oder Harnstoffderivat-haltigen Denaturierungsmittels und eines Fettalkoholpolyglykolethers, welcher mehr als 4 und weniger als 7 Ethoxy-Einheiten, insbesondere 5 bis 6 Ethoxy-Einheiten enthält, zur Vermeidung von Lipämie- und Gammopathie-Störungen bei der photometrischen Bestimmung von Eisen in einer Probe.

8. Reagenzienkombination zur Bestimmung von Eisen in Serum, gekennzeichnet durch
ein erstes Reagenz, enthaltend 1 bis 8 mol/l Denaturierungsmittel und mindestens 10 g/l Fettalkoholpolyglykolether welcher mehr als 4 und weniger als 7 Ethoxy-Einheiten, insbesondere 5 bis 6 Ethoxy-Einheiten enthält, und getrennt davon
ein zweites Reagenz, enthaltend 0,5 bis 50 mmol/l Farbstoff
in Form wässriger Lösungen oder zu deren Herstellung geeigneter Trockengemische.

9. Reagenzienkombination nach Anspruch 8, dadurch gekennzeichnet, daß in dem zweiten Reagenz 40 bis 200 mmol/l Reduktionsmittel enthalten sind.

## Claims

1. Method for the determination of iron in serum with release of the bound iron by addition of a protein denaturing agent, reduction of the released iron to Fe²⁺, addition of a colour reagent solution and photometric measurement of the colour complex that forms, wherein a denaturing agent containing urea or a urea derivative and a fatty alcohol polyglycol ether which contains more than 4 and less than 7 ethoxy units, in particular 5 to 6 ethoxy units are added to the serum.

2. Method as claimed in one of the previous claims, wherein the fatty alcohol polyglycol ether contains a branched or unbranched fatty alcohol residue with preferably 9 - 11 carbon atoms.

3. Method as claimed in one of the previous claims, wherein the fatty alcohol polyglycol ether is used at a concentration of at least 10 and in particular 50 to 100 g/l.

4. Method as claimed in one of the previous claims, wherein a guanidinium salt is used as the denaturing agent.

5. Method as claimed in one of the previous claims, wherein ascorbic acid, dithionite or hydroxylamine is used to reduce the iron.

6. Method as claimed in one of the previous claims, wherein bathophenanthroline, ferene or ferrozine is used as the colour reagent.

7. Use of a mixture of a denaturing agent containing urea or a urea derivative and of a fatty alcohol polyglycol ether which contains more than 4 and less than 7 ethoxy units, in particular 5 to 6 ethoxy units to avoid interference by lipaemia and gammopathy in the photometric determination of iron in a sample.

8. Combination of reagents for the determination of iron in serum, characterized by
a first reagent containing 1 to 8 mol/l denaturing agent and at least 10 g/l fatty alcohol polyglycol ether which contains more than 4 and less than 7 ethoxy units, in particular 5 to 6 ethoxy units and separate therefrom
a second reagent containing 0.5 to 50 mmol/l dye
in the form of aqueous solutions or dry mixtures suitable for their preparation.

9. Combination of reagents as claimed in claim 8, wherein the second reagent contains 40 to 200 mmol/l reducing agent.

## Revendications

1. Procédé pour la détermination du fer dans du sérum avec libération du fer lié, par addition d'un agent de dénaturation de protéines, réduction du fer libéré en Fe²⁺, addition d'une solution réactive de coloration et mesure photométrique du complexe coloré formé, caractérisé en ce qu'on ajoute au sérum un agent de dénaturation contenant de l'urée ou un dérivé de l'urée et un polyglycoléther d'alcool gras, qui comprend plus de 4 et moins de 7 unités éthoxy, en particulier 5 à 6 unités éthoxy.

2. Procédé selon l'une quelconque des revendications précédentes, caractérisé en ce que le polyglycoléther d'alcool gras contient un résidu d'alcool gras ramifié ou non ramifié comprenant de préférence 9 à 11 atomes de carbone.

3. Procédé selon l'une quelconque des revendications précédentes, caractérisé en ce que le polyglycoléther d'alcool gras est utilisé en une concentration d'au moins 10 g/l, en particulier de 50 à 100 g/l.

4. Procédé selon l'une quelconque des revendications précédentes, caractérisé en ce qu'on utilise un sel de guanidinium comme agent de dénaturation.

5. Procédé selon l'une quelconque des revendications précédentes, caractérisé en ce qu'on utilise de l'acide ascorbique, du dithionite ou de l'hydroxylamine pour la réduction du fer.

6. Procédé selon l'une quelconque des revendications précédentes, caractérisé en ce qu'on utilise du bathophénanthroline, du férène ou du ferrozine comme réactif colorant.

7. Utilisation d'un mélange d'un agent de dénaturation contenant de l'urée ou un dérivé de l'urée et d'un polyglycoléther d'alcool gras, qui contient plus de 4 et moins de 7 unités éthoxy, en particulier 5 à 6 unités éthoxy, pour éviter des perturbations de lipémie et de gammapathie lors de la détermination photométrique du fer dans un échantillon.

8. Combinaison de réactifs pour la détermination de fer dans du sérum, caractérisé par
un premier réactif, contenant 1 à 8 moles/l d'agent de dénaturation et au moins 10 g/l de polyglycoléther d'alcool gras, qui contient plus de 4 et moins de 7 unités éthoxy, en particulier 5 à 6 unités éthoxy, et, séparé de celui-ci,
un deuxième réactif, contenant 0,5 à 50 mmoles/l de colorant,
sous forme de solutions aqueuses ou des mélanges secs appropriés pour sa préparation.

9. Combinaison de réactifs selon la revendication 8, caractérisé en ce que le deuxième réactif contient 40 à 200 mmoles/l d'agent de réduction.
